# EUROPEAN PATENT APPLICATION

(11) **EP 4 023 212 A1**
(43) Date of publication of application: **06.07.2022**
(21) Application number: 20856835.2
(22) Date of filing: 27.08.2020
(51) Int. Cl.: A61K 9/14, A61K 31/4406, A61K 47/14, A61P 7/00, A61P 31/12, A61P 35/00, A61P 37/00

(54) **CHIDAMIDE PHARMACEUTICAL COMPOSITION, PREPARATION METHOD THEREFOR AND APPLICATION THEREOF**

(30) Priority: 28.08.2019 CN 201910803057
(71) Applicant: Shenzhen Chipscreen Biosciences Co., Ltd., Shenzhen, Guangdong 518057 (CN)
(72) Inventor: LU, Xianping, Shenzhen, Guangdong 518057 (CN); WANG, Shigang, Shenzhen, Guangdong 518057 (CN); SHAN, Song, Shenzhen, Guangdong 518057 (CN); ZHAO, Chuantong, Shenzhen, Guangdong 518057 (CN); ZHANG, Yu, Shenzhen, Guangdong 518057 (CN); DENG, Xingyu, Shenzhen, Guangdong 518057 (CN); PAN, Desi, Shenzhen, Guangdong 518057 (CN)
(74) Representative: Colombo, Stefano Paolo
(86) International application number: PCT/CN2020/111540
(87) International publication number: WO 2021/037091

(57) **Abstract**

A chidamide pharmaceutical composition, a preparation method therefor and an application thereof. The pharmaceutical composition comprises chidamide and a pharmaceutically acceptable enteric excipient that severe as a carrier. In the pharmaceutical composition, chidamide is used as a guest molecule, and the pharmaceutically acceptable enteric excipient is used as a carrier molecule. Oral pharmacokinetic testing on animal proves that the bioavailability of the composition comprising chidamide and the pharmaceutically acceptable enteric excipient is greatly improved, while adverse reactions caused by the drug in the gastrointestinal tract are reduced; in addition, the dosage may be reduced while maintaining the curative effect, thus having more important clinical significance than commercially available chidamide tablets.

## Description

This application is based on the CN Application No. 201910803057.8 with application date of August 28, 2019, and claims its priority. The disclosure of the CN application is hereby incorporated into this application in its entirety.

### Technical Field

The present invention relates to the technical field of pharmaceutical technology, in particular to a chidamide pharmaceutical composition as well as a preparation method and application thereof.

### Background Art

Chidamide is a new molecular entity drug exclusively discovered by Shenzhen Microchip Biotechnology Co., Ltd., it has a novel action mechanism and is the world's first subtype selective histone deacetylase (HDAC) inhibitor and the world's first approved oral drug for the treatment of peripheral T-cell lymphoma, and it belongs to epigenetic regulator drugs.

Chidamide has a very small solubility in water and a low bioavailability, which causes the disadvantages of poor drug absorption, high dosage and high gastrointestinal toxicity. Therefore, it is of great significance to improve the bioavailability of chidamide so as to reduce drug dosage, reduce drug production cost and reduce gastrointestinal toxicity.

CN201310364845.4 discloses a solid dispersion preparation of chidamide, comprising active component and polyvinylpyrrolidone (PVP). It is administered orally for convenience, but the solubility, stability and bioavailability of chidamide are not studied and improved.

CN201410016221.8 discloses a solid dispersion of chidamide and its preparation method and application, and the preferred preparation is the tablet of solid dispersion made of chidamide and povidone K30 with a weight ratio of 1:5. In the solid dispersion, chidamide can be highly dispersed in water-soluble carrier material in molecular form or amorphous state. The results show that the solid dispersion has a solubility of 66.7 µg/ml and a dissolution of 79.1% in water. The commercial chidamide solid dispersion tablets is just this preparation.

CN201610855106.9 discloses a solid dispersion of benzamide compound in E configuration, in which by combining chidamide with copovidone (especially in a weight ratio of 1:2 to 1:10), the compound of Formula (I) can be highly dispersed in copovidone in molecular form or amorphous state, it improves the water solubility and dissolution rate of the compound of Formula (I), and also improves the stability of the formed solid dispersion.

There is still a huge demand in this field for obtaining a chidamide preparation with higher bioavailability.

### Contents of the present invention

In view of this, the object of the present invention is to provide a chidamide pharmaceutical composition and a preparation method thereof, so as to improve the bioavailability of chidamide. The pharmaceutical composition and the preparation method can be applied to the fields of drug preparation and disease treatment related to chidamide.

The term "carrier" as used herein refers to a material used for embedding chidamide, which can dissolve or disperse chidamide in the material.

The term "solid dispersion" used herein refers to a dispersion system in solid form formed by the uniform dispersion or distribution of a drug in a highly dispersed state such as molecular, amorphous or microcrystalline state in a dispersion medium (e.g., a carrier described herein).

In order to achieve the above object of the present invention, the present invention provides the following technical solution:
In one aspect, the present invention provides a chidamide pharmaceutical composition comprising chidamide and a pharmaceutically acceptable enteric excipient as a carrier.

The chidamide of the present invention has the structure shown in Formula (1), and its chemical name is N-(2-amino-4-fluorophenyl)-4-[N-[(E)-3-(3-pyridyl)acryloyl]aminomethyl]benzamide. In its structural formula, 3-pyridylacryloyl is of E configuration.

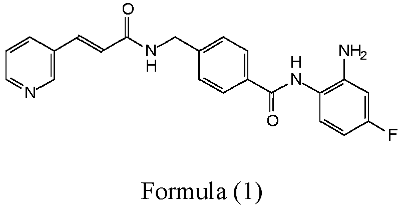

In some embodiments, the present invention provides a pharmaceutically acceptable enteric excipient with better effect for improving bioavailability, which is at least one or two selected from the group consisting of hydroxypropyl methylcellulose acetate succinate, cellulose acetate phthalate, polyvinyl alcohol phthalate, cellulose acetate trimellitate, hypromellose phthalate and acrylic resin.

In some embodiments, the mass ratio of chidamide to the pharmaceutically acceptable enteric excipient ranges from 1:1 to 1:100, more preferably from 1:1 to 1:20, most preferably from 1:1 to 1:10, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90 or 1:100. In some embodiments, the mass ratio is 1:3, 1:5, 1:8, 1:50 or 1:100.

In the present invention, a solid dispersion prepared by using a conventional pharmaceutically acceptable excipient for chidamide preparations polyvinylpyrrolidone K30 (K30 has the function of improving bioavailability in other drugs) as carrier, is taken as a control to compare with the chidamide pharmaceutical composition of the present invention in terms of bioavailability. The results show that, the AUC (area under the drug time curve) of the solid dispersion is only about 60% of the AUC of the pharmaceutical composition of the present invention, the maximum concentration of the former is only 836.1 ng/ml, while the maximum concentration of the pharmaceutical composition of the present invention can reach 2059.9 ng/ml. On the other hand, compared with the commercially available chidamide solid dispersion, plasma concentrations at different time points of the pharmaceutical composition of the present invention are all significantly higher than those of the commercially available drug.

On the basis of the aforementioned significant technical advantages, the present invention provides use of the pharmaceutical composition in the manufacture of a chidamide preparation and/or a medicament for the treatment of a disease associated with the action mechanism of histone deacetylase.

In some embodiments, the disease associated with the action mechanism of histone deacetylase is selected from the group consisting of cancer, viral disease, autoimmune disease and blood system disease.

On the basis of the aforementioned application, another aspect of the present invention also provides a chidamide preparation, comprising the chidamide pharmaceutical composition of the present invention. In some embodiments, based on the total weight of all substances comprised in the preparation, the preparation comprises 20% to 60% of the aforementioned chidamide pharmaceutical composition, for example, 20%, 25%, 30%, 35%, 40%, 45%, 50%, 55% or 60% by weight.

In some embodiments, the preparation comprises the aforementioned chidamide pharmaceutical composition and a pharmaceutically acceptable excipient. In some embodiments, the preparation is a tablet, capsule, pill, oral liquid preparation, granule, powder, plaster or dropping pill. In some embodiments, the preparation can be prepared into a dosage form of any release form such as a sustained-release preparation, controlled-release preparation, etc.

The pharmaceutically acceptable excipient can be adjusted and selected according to the dosage form to be prepared. In the present invention, the excipient can be at least one or two selected from the group consisting of fillers, disintegrating agents, binding agents and lubricants. The amount of the fillers can be 0% to 90%, preferably 30% to 80%, for example, 40% to 80%, 5% to 20%, and for further example, 5%, 10%, 15%, 20%, 25%, 30%, 35%, 40%, 45%, 50%, 55%, 60%, 65%, 70%, 75% or 80%; the amount of the disintegrating agents can be 0% to 10%, preferably 3% to 8%, for example, 0.5% to 6%, and for further example, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10%; the amount of the binding agents can be 0% to 20%, preferably 0% to 5%, for example, 0%, 0.5%, 1%, 1.5%, 2%, 2.5%, 3%, 3.5%, 4%, 4.5% or 5%; the amount of pH regulators can be 0% to 20%, preferably 0% to 5%; the amount of the lubricants can be 0% to 5%, preferably 0.2% to 1%, for example, 0%, 0.5%, 1%, 1.5%, 2%, 2.5%, 3%, 3.5%, 4%, 4.5% or 5%.

In some embodiments, the fillers is selected from at least one or two of lactose, corn starch, hypromellose, hydroxypropyl cellulose, cetyl alcohol, octadecyl alcohol, ethyl cellulose, pregelatinized starch, sucrose, microcrystalline cellulose, mannitol, calcium hydrogen phosphate, calcium phosphate, xanthan gum and colloidal silica;
the disintegrating agents is selected from at least one or two of starch, microcrystalline cellulose, croscarmellose, sodium carboxymethyl cellulose, sodium carboxymethyl starch, crosslinked polyvinylpyrrolidone and low substituted hydroxypropyl cellulose;
the binding agents is selected from at least one or two of water, ethanol, hydroxypropyl cellulose, hypromellose, hydroxyethyl cellulose, polyvinylpyrrolidone and copovidone;

The lubricants is selected from at least one or two of stearic acid, magnesium stearate, sodium stearylfumarate, polyethylene glycol 4000-8000, aerosil and talc powder.

In some embodiments, the chidamide preparation is a capsule. Based on the total weight of the capsule, the capsule comprises:
i) 25% to 35% by weight of the pharmaceutical composition described herein;
ii) 62% to 72% by weight of a filler;
iii) 1% to 3% by weight of a disintegrating agent; and
iv) 1% to 2% by weight of a lubricant;
or,
25% to 35% by weight of the pharmaceutical composition described herein and a blank pellet; preferably, the blank pellet is a microcrystalline cellulose blank pellet.

In some embodiments, the chidamide preparation is a capsule, and based on the total weight of the capsule, for each 1000 capsules, the capsule comprises:

| | |
|---|---|
| chidamide | 5g |
| acrylic resin L100-55 | 15g |
| mannitol | 20g |
| microcrystalline cellulose | 35g |
| croscarmellose | 2g |
| hypromellose | 2g; and |
| magnesium stearate | 1g. |

In some embodiments, the chidamide preparation is a capsule, and based on the total weight of the capsule, for each 1000 capsules, the capsule comprises:

| | |
|---|---|
| chidamide | 5g; |
| hypromellose acetate succinate | 40g; and |
| microcrystalline cellulose blank pellet | 100g. |

In some embodiments, the chidamide preparation is a tablet, and based on the total weight of the tablet, the tablet comprises:
i) 20% to 50% by weight of the pharmaceutical composition described herein;
ii) 40% to 70% by weight of a filler;
iii) 3% to 8% by weight of a disintegrating agent;
iv) 0.5% to 2% by weight of a lubricant;
v) 0% to 5% by weight of a binding agent.

In some embodiments, the chidamide preparation is a tablet, and based on the total weight of the tablet, for each 1000 tablets, the tablet comprises:

| | |
|---|---|
| chidamide | 5g; |
| hypromellose phthalate | 25g; |
| mannitol | 40g; |
| microcrystalline cellulose | 51g; |
| sodium carboxymethyl starch | 5g; |
| polyvinylpyrrolidone | 3g; and |
| magnesium stearate | 1g |

In some embodiments, the chidamide preparation is a tablet, and based on the total weight of the tablet, for each 1000 tablets, the tablet comprises:

| | |
|---|---|
| chidamide | 5g; |
| cellulose acetate trimellitate | 50g; |
| calcium hydrogen phosphate | 15g; |
| corn starch | 40g; |
| crospovidone | 5g; |
| hydroxyethyl cellulose | 3g; |
| sodium stearyl fumarate | 1g; |
| Eudragit L100-55 | 4.5g; |
| triethyl citrate | 0.45g; and |
| talc powder | 1.1g. |

In some embodiments, the chidamide preparation is a granule, and based on the total weight of the granule, the granule comprises:
i) 50% to 60% by weight of the pharmaceutical composition described herein;
ii) 30% to 50% by weight of a filler; and
iii) 1% to 5% by weight of a disintegrating agent.

In some embodiments, the chidamide preparation is a granule, and based on the total weight of the granule, for each 1000 granules, the granule comprises:

| | |
|---|---|
| chidamide | 5g; |
| polyvinyl alcohol phthalate | 250g; |
| lactose | 200g; |
| hydroxypropyl cellulose | 10g; |
| low-substituted sodium hydroxypropyl cellulose | 5g; and |
| silica | 2g. |

In some embodiments, the chidamide preparation is a dry suspension, and based on the total weight of the dry suspension, the dry suspension comprises:
i) 40% to 50% by weight of the pharmaceutical composition described herein;
ii) 40% to 60% by weight of a filler; and
iii) 0.2% to 1% by weight of a disintegrating agent.

In some embodiments, the chidamide preparation is a dry suspension, and based on the total weight of the dry suspension, for each 1000 pills, the dry suspension comprises:

| | |
|---|---|
| chidamide | 5g; |
| cellulose acetate phthalate | 500g; |
| mannitol | 500g; |
| sodium carboxymethyl cellulose | 5g; |
| xanthan gum | 5g; and |
| silica | 5g. |

In addition, in another aspect, the present invention also provides a method for preparing the pharmaceutical composition, comprising a step of preparing the pharmaceutical composition with chidamide and the pharmaceutically acceptable enteric excipient described herein by a solvent evaporation method or a hot melt extrusion method.

In some embodiments, the present invention adopts a solvent evaporation method to prepare the pharmaceutical composition, wherein the solvent is selected from pharmaceutically acceptable alcohol, acetone, tetrahydrofuran or any combination thereof.

In some embodiments, the alcohol is selected from at least one or two of methanol, ethanol, isopropanol, butanol, tert-butanol, and propanol.

In some specific embodiments, the preparation method of the pharmaceutical composition is: dissolving chidamide and the pharmaceutically acceptable enteric excipient in a solvent, and subjecting the obtained solution to rotary evaporation or spray drying.

In another aspect, the present invention provides use of the chidamide pharmaceutical composition or chidamide preparation in the manufacture of a HDAC inhibitor.

In another aspect, the present invention provides a method of inhibiting HDAC, comprising a step of administering to a subject in need thereof an effective amount of the chidamide pharmaceutical composition or chidamide preparation described herein.

In another aspect, the present invention provides a method of treating a disease associated with the action mechanism of histone deacetylase, comprising a step of administering to a subject in need thereof an effective amount of the chidamide pharmaceutical composition or chidamide preparation described herein.

In some embodiments, the disease associated with the action mechanism of histone deacetylase is selected from cancer, viral disease, autoimmune disease and blood system disease.

As can be seen from the above technical solutions, the present invention provides a pharmaceutical composition comprising chidamide and a specific pharmaceutically acceptable enteric excipient, in which chidamide is used as a guest molecule and the pharmaceutically acceptable enteric excipient is used as a carrier molecule. Oral pharmacokinetic testing on animal proves that the bioavailability of the composition comprising chidamide and the pharmaceutically acceptable enteric excipient is greatly improved, while adverse reactions caused by the drug in the gastrointestinal tract are reduced; in addition, the dosage may be reduced while maintaining the curative effect, thus having more important clinical significance than commercially available chidamide tablets.

### Brief Description of the Drawings

Figure 1 shows the results of the rat pharmacokinetic experiment of the chidamide pharmaceutical composition;
Figure 2 shows the mean plasma concentration-time curves in dog plasma for commercially available chidamide tablet, Example 4 chidamide tablet, Example 6 chidamide capsule, and Example 8 chidamide capsule.

### Specific Modes for Carrying Out the invention

The present invention discloses a chidamide pharmaceutical composition and a preparation method and application thereof, which can be achieved by those skilled in the art by learning from the content described herein and appropriately improving process parameters. It should be particularly pointed out that all similar substitutions and modifications are obvious to those skilled in the art, and they are deemed to be included in the present invention. The pharmaceutical composition of the present invention and its preparation method and application have been described through the preferred examples, and relevant persons can obviously realize and apply the technology of the present invention by changing or making appropriate modification and combination of the pharmaceutical composition and its preparation method described herein without departing from the content, spirit and scope of the present invention.

The chidamide pharmaceutical composition, its preparation method and application provided by the present invention are further elucidated below.

### Example 1: Chidamide solid dispersion preparation as control

Chidamide solid dispersion was prepared by taking polyvinylpyrrolidone K30 as a carrier, wherein the ratio of chidamide to polyvinylpyrrolidone K30 was 1:5, and the preparation method of chidamide solid dispersion was as follows:
prescription:

| | |
|---|---|
| chidamide | 2g |
| polyvinylpyrrolidone K30 | 10g |
| ethanol | an appropriate amount |

the preparation method was as follows:
(1) chidamide and polyvinylpyrrolidone K30 were accurately weighed, dissolved in an appropriate amount of ethanol in a 90°C water bath to form a transparent solution;
(2) the solution formed in step (1) was rotary-evaporated at 90°C and dried to form a solid dispersion;
(3) the solid dispersion obtained in step (2) was taken out and pulverized to prepare a chidamide solid dispersion powder.

### Example 2: Chidamide pharmaceutical composition of the present invention

Chidamide pharmaceutical composition was prepared by using hypromellose acetate succinate as a carrier, wherein the ratio of chidamide to hypromellose acetate succinate was 1:3, and the preparation method of the chidamide pharmaceutical composition was as follows:
prescription:

| | |
|---|---|
| chidamide | 2g |
| hypromellose acetate succinate | 6g |
| ethanol | an appropriate amount |

the preparation method was as follows:
(1) chidamide and hypromellose acetate succinate were accurately weighed, dissolved in an appropriate amount of ethanol in a 90°C water bath to form a transparent solution;
(2) the solution formed in step (1) was rotary-evaporated at 90°C and dried to form a solid dispersion;
(3) the solid dispersion obtained in step (2) was taken out and pulverized to prepare a chidamide pharmaceutical composition powder.

### Example 3: Rat pharmacokinetic experiment

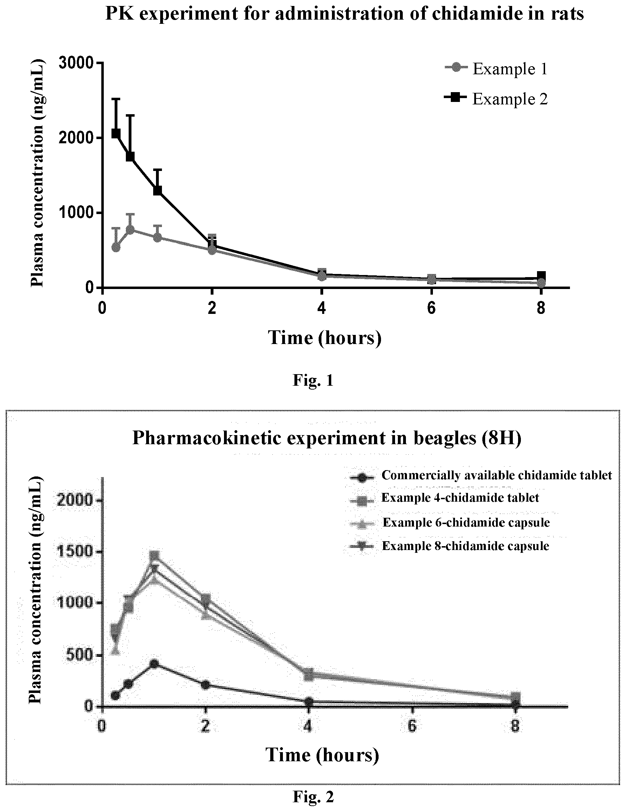

12 healthy rats were selected and randomly divided into 2 groups, 6 rats in each group (3 females and 3 males), namely Chidamide solid dispersion group, Chidamide-Hypromellose acetate succinate (1:3) group. During the experiment, the rats were fasted overnight before administration, and blood was collected from the orbit before administration, and the plasma was separated, which was taken as the plasma concentration sample at 0 h. Chidamide solid dispersion prepared in Example 1 and Chidamide-Hypromellose acetate succinate (1:3) prepared in Example 2 were dispersed with purified water respectively to give gavage solution, the concentration of Chidamide in each gavage solution was 2 mg/ml, and each rat was administered with chidamide 20 mg/kg respectively. After administration, blood samples were collected at 15min, 0.5h, 1h, 2h, 4h, 6h, and 8h, and each sample collected was about 0.5ml; the samples were anticoagulated with heparin sodium, placed on ice after collection, and centrifuged within 1 hour to separate plasma which was then stored at -80°C for later testing. The drug concentration in plasma was measured by LC-MS/MS. The test results were shown in Table 1 and Figure 1.

**Table 1: Comparison of pharmacokinetic data in rats**

| Parameter (ng/ml) | Example 1 | Example 2 |
|---|---|---|
| AUC(0-t) | 2271.4 | 3716.4 |
| Cmax | 836.1 | 2059.9 |

According to the test results in Table 1 and Figure 1, compared with the chidamide solid dispersion tablet, the chidamide pharmaceutical composition comprising hypromellose acetate succinate showed a significantly improved bioavailability of chidamide.

### Example 4: Tablet preparation comprising the chidamide pharmaceutical composition of the present invention

Chidamide pharmaceutical composition was prepared by using hypromellose phthalate as a carrier, wherein the ratio of chidamide to hypromellose phthalate was 1:5, and the chidamide tablet preparation and the preparation method thereof were as follows:
prescription:

| | | |
|---|---|---|
| chidamide | | 5g |
| hypromellose phthalate | | 25g |
| mannitol | | 40g |
| microcrystalline cellulose | | 51g |
| sodium carboxymethyl starch | | 5g |
| polyvinylpyrrolidone | | 3g |
| magnesium stearate | | 1g |
| ethanol | | an appropriate amount |
| water | | an appropriate amount |
| made into | 1000 tablets (pills) | |

preparation method:
(1) chidamide and hypromellose phthalate were accurately weighed and dissolved in ethanol to form a transparent solution;
(2) the solution obtained in step (1) was spray-dried to obtain a chidamide-hypromellose phthalate composition;
(3) the composition obtained in step (2) was uniformly mixed with mannitol, microcrystalline cellulose, sodium carboxymethyl starch, and polyvinylpyrrolidone, added with water and subjected to wet granulation to obtain a soft material;
(4) the soft material obtained in step (3) was dried to obtain dry particles;
(5) the dry particles obtained in step (4) were added with magnesium stearate to perform total mixing;
(6) the material of total mixing obtained in step (5) was subjected to tableting to obtain the tablet preparation.

### Example 5: Granule preparation comprising the chidamide pharmaceutical composition of the present invention

Chidamide pharmaceutical composition was prepared by using polyvinyl alcohol phthalate as a carrier, wherein the ratio of chidamide to polyvinyl alcohol phthalate was 1:50, and the chidamide granule preparation and the preparation method thereof were as follows:
prescription:

| | | |
|---|---|---|
| chidamide | | 5g |
| polyvinyl alcohol phthalate | | 250g |
| lactose | | 200g |
| hydroxypropyl cellulose | | 10g |
| Low-substituted hydroxypropyl cellulose sodium | | 5g |
| silica | | 2g |
| tetrahydrofuran/ethanol (1:1) | | an appropriate amount |
| water | | an appropriate amount |
| made into | 1000 tablets (pills) | |

preparation method:
(1) chidamide and polyvinyl alcohol phthalate were accurately weighed and dissolved in tetrahydrofuran/ethanol (1:1) to form a solution;
(2) the solution obtained in step (1) was spray-dried to obtain a chidamide-polyvinyl alcohol phthalate composition;
(3) the composition obtained in step (2) was uniformly mixed with lactose, hydroxypropyl cellulose and low-substituted hypromellose, added with water to obtain a soft material, and subjected to a shaking method to obtain particles;
(4) the particles obtained in step (3) were dried to obtain dry particles;
(5) the dry particles obtained in step (4) were added with silica, and mixed uniformly;
(6) the mixture obtained in step (5) was bagged to obtain the granule preparation.

### Example 6: Capsule preparation comprising the chidamide pharmaceutical composition of the present invention

Chidamide pharmaceutical composition was prepared by using acrylic resin L100-55 as a carrier, wherein the ratio of chidamide to acrylic resin L100-55 was 1:3, and the chidamide capsule preparation and the preparation method thereof were as follows:
prescription:

| | | |
|---|---|---|
| chidamide | | 5g |
| acrylic resin L100-55 | | 15g |
| mannitol | | 20g |
| microcrystalline cellulose | | 35g |
| croscarmellose sodium | | 2g |
| hypromellose | | 2g |
| magnesium stearate | | 1g |
| acetone | | an appropriate amount |
| water | | an appropriate amount |
| made into | 1000 tablets (pills) | |

preparation method:
(1) chidamide and acrylic resin L100-55 were accurately weighed, and dissolved in acetone to form a transparent solution;
(2) the solution obtained in step (1) was subjected to vacuum rotary-evaporation to obtain a chidamide-acrylic resin L100-55 composition;
(3) the composition obtained in step (2) was mixed with mannitol, microcrystalline cellulose, croscarmellose sodium, and hypromellose uniformly, added with water to obtain a soft material, and subjected to a shaking method to obtain particles;
(4) the particles obtained in step (3) were dried to obtain dry particles;
(5) the dry particles obtained in step (4) were added with magnesium stearate, and subjected to total mixing;
(6) the material of the total mixing obtained in step (5) was filled into capsules to obtain the capsule preparation.

### Example 7: Dry suspension preparation comprising the chidamide pharmaceutical composition of the present invention

Chidamide pharmaceutical composition was prepared by using cellulose acetate phthalate as a carrier, wherein the ratio of chidamide to cellulose acetate phthalate was 1:100, and the chidamide dry suspension preparation and the preparation method thereof were as follows:
prescription:

| | | |
|---|---|---|
| chidamide | | 5g |
| cellulose acetate phthalate | | 500g |
| mannitol | | 500g |
| sodium carboxymethyl cellulose | | 5g |
| xanthan gum | | 5g |
| silica | | 5g |
| acetone | | an appropriate amount |
| water | | an appropriate amount |
| made into | 1000 tablets (pills) | |

preparation method:
(1) chidamide and cellulose acetate phthalate were accurately weighed and dissolved in ethanol to form a transparent solution;
(2) the solution obtained in step (1) was spray-dried to obtain a chidamide-cellulose acetate phthalate composition;
(3) under stirring conditions, sodium carboxymethyl cellulose was added into purified water, and continuously stirred until completely dissolved;
(4) the composition obtained in step (2) was mixed uniformly with mannitol, then added into the sodium carboxymethyl cellulose solution obtained in step (3), and subjected to wet granulation;
(5) the particles obtained in step (4) were dried to obtain dry particles;
(6) the dry particles obtained in step (5) were added with xanthan gum and silica, and mixed for breaking;
(7) the dry suspension powder obtained in step (6) was subjected to subpackaging.

### Example 8: Capsule preparation comprising the chidamide pharmaceutical composition of the present invention

The chidamide pharmaceutical composition was prepared by using hypromellose acetate succinate as a carrier, wherein the ratio of chidamide to hypromellose acetate succinate was 1:8, and the chidamide capsule preparation and the preparation method thereof were as follows:
prescription:

| | | |
|---|---|---|
| chidamide | | 5g |
| hypromellose acetate succinate | | 40g |
| microcrystalline cellulose blank pellets | | 100g |
| made into | 1000 tablets (pills) | |

preparation method:
(1) chidamide and hypromellose acetate succinate were accurately weighed, and dissolved in ethanol to form a transparent solution;
(2) the solution obtained in step (1) was loaded on the microcrystalline cellulose blank pellets by using a fluidized bed to obtain chidamide-containing pellets;
(3) the pellets obtained in step (2) were filled into capsules to obtain the capsule preparation.

### Example 9: Tablet preparation comprising the chidamide pharmaceutical composition of the present invention

Chidamide pharmaceutical composition was prepared by using cellulose acetate trimellitate as a carrier, wherein the ratio of chidamide to cellulose acetate trimellitate was 1:10, and the chidamide tablet preparation and the preparation method thereof were as follows:
prescription:

| | | |
|---|---|---|
| chidamide | | 5g |
| cellulose acetate trimellitate | | 50g |
| calcium hydrogen phosphate | | 15g |
| corn starch | | 40g |
| crospovidone | | 5g |
| hydroxyethyl cellulose | | 3g |
| sodium stearyl fumarate | | 1g |
| Eudragit L100-55 | | 4.5g |
| triethyl citrate | | 0.45g |
| talc powder | | 1.1g |
| ethanol | | an appropriate amount |
| water | | an appropriate amount |
| made into | 1000 tablets (pills) | |

preparation method:
(1) chidamide and cellulose acetate trimellitate were accurately weighed, and mixed evenly;
(2) the mixture obtained in step (1) was subjected to hot-melt extrusion to prepare a chidamide-hypromellose phthalate composition;
(3) the composition obtained in step (2) was uniformly mixed with calcium hydrogen phosphate, corn starch, crospovidone, and hydroxyethyl cellulose, and added with water to carry out wet granulation to obtain a soft material;
(4) the soft material obtained in step (3) was dried to obtain dry particles;
(5) the dry particles obtained in step (4) were added with sodium stearyl fumarate, and subjected to total mixing;
(6) the material of the total mixing obtained in step (5) was subjected to tabletting to obtain a tablet preparation;
(7) Eudragit L100-55, triethyl citrate and talc powder were added into an appropriate amount of ethanol, and stirred well to prepare an enteric coating solution;
(8) the tablet preparation obtained in step (6) was coated by the enteric coating solution obtained in step (7) to obtain chidamide enteric-coated tablets.

### Example 10: Pharmacokinetic study in beagle dogs

Twelve healthy beagle dogs were selected and randomly divided into four groups, three dogs in each group, namely Commercially available chidamide tablet group, Example 1 chidamide tablet group, Example 3 chidamide capsule group and Example 5 chidamide capsule group. During the experiment, beagle dogs were fasted overnight before administration, and venous blood was collected before administration, and plasma was separated as the plasma concentration sample at 0h. Each dog was administered with chidamide 20 mg (4 pills, 5 mg/pill). After administration, blood samples were collected at 15min, 0.5h, 1h, 2h, 4h, and 8h respectively, each sample collected was about 0.5ml, the samples were anticoagulated with heparin sodium, placed on ice after collection, and centrifuged within 1 hour to separate plasma which was then measured by LC-MS/MS to determine drug concentration in plasma. The test results were shown in Table 2 and Figure 2.

**Table 2: LC-MS/MS determination of drug concentrations (ng/ml) in dog plasma for each group at different time points**

| Time (h) | Commercially available tablet | Example 4 tablet | Example 6 capsule | Example 8 capsule |
|---|---|---|---|---|
| 0.25 | 110.291 | 754.028 | 555.006 | 655.453 |
| 0.50 | 222.878 | 953.046 | 1021.364 | 1036.435 |
| 1.00 | 416.646 | 1461.740 | 1236.307 | 1332.347 |
| 2.00 | 213.347 | 1049.735 | 891.904 | 966.674 |
| 4.00 | 50.908 | 298.286 | 336.756 | 327.331 |
| 8.00 | 19.411 | 95.123 | 71.706 | 74.923 |

According to the test results in Table 2 and Figure 2, compared with the commercially available chidamide solid dispersion tablet (registered trademark: Epidaza), after chidamide and the pharmaceutically acceptable excipient of the present invention formed a composition, the bioavailability of chidamide in vivo was effectively improved.

The above are only the preferred embodiments of the present invention. It should be pointed out that for those skilled in the art, without departing from the principle of the present invention, several improvements and modifications can be made, and these improvements and modifications should be regarded as falling within the scope of the present invention.

## Claims

1. A chidamide pharmaceutical composition, comprising chidamide and a pharmaceutically acceptable enteric excipient as a carrier.

2. The pharmaceutical composition according to claim 1, wherein the pharmaceutically acceptable enteric excipient is selected from one or more than two of hypromellose acetate succinate, cellulose acetate phthalate, polyvinyl alcohol phthalate, cellulose acetate trimellitate, hypromellose phthalate and acrylic resin.

3. The pharmaceutical composition according to claim 1 or 2, wherein the mass ratio of chidamide to the pharmaceutically acceptable enteric excipient ranges from 1:1 to 1:100.

4. A chidamide preparation, comprising the chidamide pharmaceutical composition according to any one of claims 1 to 3.

5. The preparation according to claim 4, which is a tablet, capsule, pill, oral liquid preparation, granule, powder, plaster or dropping pill.

6. Use of the pharmaceutical composition according to any one of claims 1 to 3 in the manufacture of a chidamide preparation.

7. Use of the pharmaceutical composition according to any one of claims 1 to 3 or the chidamide preparation according to claim 5 or 6 in the manufacture of a medicament for the treatment of a disease associated with the action mechanism of histone deacetylase.

8. The use according to claim 7, wherein the disease associated with the action mechanism of histone deacetylase includes cancer, viral disease, autoimmune disease and blood system disease.

9. A method for preparing the pharmaceutical composition according to any one of claims 1 to 3, comprising a step of preparing the pharmaceutical composition with chidamide and the pharmaceutically acceptable enteric excipient by a solvent evaporation method or hot melt extrusion method.

10. The method according to claim 9, wherein the solvent used in the solvent volatilization method is selected from pharmaceutically acceptable alcohol, acetone, tetrahydrofuran or any combination thereof.

11. The method according to claim 10, wherein the alcohol is selected from at least one or two of methanol, ethanol, isopropanol, butanol, tert-butanol and propanol.
